# EUROPÄISCHE PATENTSCHRIFT

(11) **EP 1 886 686 B1**
(45) Veröffentlichungstag und Bekanntmachung des Hinweises auf die Patenterteilung: **10.04.2013**
(21) Anmeldenummer: 07014491.0
(22) Anmeldetag: 24.07.2007
(51) Int. Cl.: A61K 31/58, A61K 9/00, A61K 31/57, A61K 47/10

(54) **Pharmazeutische Zusammensetzung enthaltend Mometasonfuroat**
Pharmaceutical composition containing mometasone furoate
Composition pharmaceutique comportant du mométasone furoate

(30) Priorität: 25.07.2006 DE 102006034883
(43) Veröffentlichungstag der Anmeldung: 13.02.2008
(73) Patentinhaber: Almirall Hermal GmbH, 21465 Reinbek (DE)
(72) Erfinder: Brauel, Anke, Dr., 21077 Hamburg (DE); Evers, Dirk, 21465 Reinbek (DE); Evers, Fritjof, 21465 Reinbek (DE); Fielhauer, Sabine, 21481 Lauenburg (DE); Henning, Dr., Falkenweg 9c 21244 Buchholz/N (DE); Meyer, Ricarda, 21271 Hanstedt (DE); Strahinjic, Ivana, 22307 Hamburg (DE); Treudler, Klaus, Dr., 21217 Seevetal (DE); Vollus, Marlies, 20537 Hamburg (DE)
(74) Vertreter: Polypatent

(56) Entgegenhaltungen:
- WO-A-01/26658
- WO-A-91/08733
- US-B1- 6 180 781
- US-B1- 6 479 058

## Beschreibung

Die Erfindung bezieht sich auf eine Mometasonfuroat enthaltende pharmazeutische Zusammensetzung.

Mometasonfuroat ist ein in der EP 0057401 beschriebenes entzündungshemmendes Steroid mit dem chemischen Namen 9a, 21-Dichlor-16a-methyl-1,4-pregnadien-11β, 17a-diol-3, 20-dion-17-(2'-furoat).

Mometasonfuroat gehört zu der Klasse der synthetischen topischen Corticosteroide, die bei der Behandlung entzündlichen Hauterkrankungen zum Einsatz kommen können.

Im Handel sind Mometason-haltige Produkte zur Behandlung der Haut z.B. als Salbe, Fettcreme und Lösung erhältlich.

Typische Anwendungsgebiete von Mometasonfuroat sind entzündliche Hauterkrankungen, wie z.B. die Behandlung der Schuppenflechte (Psoriasis) sowie von verschiedenen Ekzemformen wie etwa dem allergischen Kontaktekzem, dem chronischen Handekzem oder dem atopischen Ekzem

Topische Zusammensetzungen mit Mometasonfuroat zur Behandlung von Entzündungen sind z.B. in der EP 0262681 und der WO2004/105686 offenbart.

Corticosteroide sind in der Regel lipophile Substanzen und somit oft schlecht in wässriger Umgebung löslich, was die Formulierung von Produkten, die solche Substanzen enthalten, erschwert. Mometasonfuroat z.B. ist nicht löslich in Wasser, weist eine schwache Löslichkeit in Octanol auf und ist begrenzt löslich in Ethylalkohol. Ein geeignetes Lösungsmittel für Mometasonfuorat ist z.B. Propylenglykol, das in aus der WO2004/105686 bekannten Zusammensetzungen eingesetzt wird. Aber auch in Gegenwart bislang eingesetzter geeigneter Lösungsmittel fällt Mometasonfuorat aus, wenn der Wasseranteil einen bestimmten Wert übersteigt.

Kommt es zur Kristallisation der Substanz aus der gelösten Form, so kann dies eine Einschränkung der Wirksamkeit nach sich ziehen.

Derzeit sind Zubereitungen mit Mometasonfuroat mit einem erhöhten Wasseranteil, in denen der Wirkstoff gelöst vorliegt, ausschließlich durch den Einsatz höherer Konzentrationen an kurzkettigen einwertigen aliphatischen Alkoholen möglich, wie z.B. ein im Handel unter dem Namen Ecural^{®} Lösung erhältliches Produkt, das 2-Propanol enthält. Einwertige Alkohole sind nachteilig für die Therapie von Hautkrankheiten, da die Anwendung auf entzündeter und geschädigter Haut häufig zu Brennen als Nebenwirkung fuhrt. Dies wiederum führt zu einer eingeschränkten Compliance durch die Patienten und damit zu einem potenziell reduzierten Therapieerfolg.

Um diese Probleme zu vermeiden wird in der Therapie häufig auf die verfügbaren Produkte mit Mometason ohne kurzkettige einwertige aliphatische Alkohole ausgewichen. Diese enthalten in der Regel nur geringe Wassergehalte unterhalb 5% (w/w), was zu Einschränkungen bei den Einsatzmöglichkeiten dieser Formulierungen führt. So wird z.B. die Anwendung dieser Produkte in Bereichen erschwert, in denen eine Okklusion unbedingt zu vermeiden ist. Weiterhin wird u.a. die Anwendung am behaarten Kopf vom Patienten durch die schwierige nachfolgende Haarreinigung als Belastung empfunden.

Die WO-A-91/08733 offenbart Öl-in-Wasser-Emulsionen zur Verwendung in pharmazeutischen Zusammensetzungen in Form einer Creme und/oder Lotion, die neben mindestens einem lipophilen Wirkstoff, wie etwa Mometasonfuroat, enthalten: a) N-Methyl-2-Pyrrolidon, um die Passage des Wirkstoffes durch die Haut zu ermöglichen, b) eine wässrige Phase, die Wasser und Propylenglykol enthält, c) eine Ölphase, die Diethyl-Sebacat oder Mineralöl enthält, d) ein Tensidsystem, e) eine okklusive Substanz und f) ein Konservierungsmittel, wobei der pH-Wert der Basis in einem Bereich von 4 bis 7 liegt.

Aufgabe der Erfindung ist, pharmazeutische Zusammensetzungen bereitzustellen, in denen Mometasonfuroat auch in Gegenwart höherer Wasseranteile gelöst vorliegt und in denen keine kurzkettigen einwertigen aliphatischen Alkohole zum in Lösung halten des Wirkstoffs erforderlich sind.

Gelöst wird diese Aufgabe mit einer Zusammensetzung, die die Merkmale des Anspruches 1 aufweist.

Alle in der Anmeldung und den Ansprüchen enthaltenen %-Angaben sind Gewichtsprozentangaben (w/w).

Es hat sich überraschend herausgestellt, daß Mometasonfuroat in Konzentrationen zwischen 0,01 % und 0,2% (w/w) in Zusammensetzungen, die zwischen 10% und 90% (w/w) Wasser enthalten, gelöst vorliegt, wenn der Zusammensetzung eine Kombination von
i) mindestens einem aromatischen Alkohol; und
ii) mindestens einem Lösungsmittel ausgewählt aus:
   einer ersten Gruppe, zu der alkoxilierte Fettalkohole mit einer Kettenlänge von C8 - C22, alkoxilierte und nicht alkoxilierte Fettsäuren mit einer Kettenlänge von C8 - C22 und alkoxilierte und nicht alkoxilierte Carbonsäureester mit Carbonsäuren einer Kettenlänge von C6 - C22 gehören; und
iii) Hexylenglykol;
zugesetzt wird, sowie gegebenenfalls weitere Zusatzstoffe. Aromatische Alkohole sind breit eingesetzte Konservierungsmittel in Arzneimitteln und Kosmetika.

Überraschend hat sich gezeigt, dass eine Kombination eines aromatischen Alkohols mit mindestens einem der Lösungsmittel aus der ersten Gruppe und Hexylenglykol das Lösungsverhalten von Mometasonfuroat derart beeinflusst, dass diese Substanz auch in Zusammensetzungen mit gegenüber dem Stand der Technik deutlich erhöhten Wassergehalt ohne Anteile an kurzkettigen, einwertigen aliphatischen Alkoholen nicht ausfällt. Dieser Effekt war bislang im Stand der Technik nicht bekannt oder nahegelegt.

Die erfindungsgemäßen Zusammensetzungen weisen im Gegensatz zu den Produkten mit einem Anteil an einwertigen aliphatischen Alkoholen eine deutlich verbesserte Patientenakzeptanz auf. Ein Brennen als häufige Nebenwirkung tritt nicht auf.

Die erfindungsgemäß möglichen Zusammensetzungen mit erhöhtem Wasseranteil in Form von z.B. Lösungen, Emulsionen oder Mikroemulsionen stellen somit eine wesentliche Verbesserung in der Therapie von Hauterkrankungen dar.

Im Gegensatz zu den bekannten Mometasonfuorat-haltigen Salben, lassen sich mit der erfindungsgemäßen Zusammensetzung Produkte darstellen, die einer Okklusion vorbeugen und damit das Risiko von Hautatrophien bzw. systemischer Resorption senken, bei erheblich reduzierten subjektiven Nebenwirkungen und einer deutlich verbesserten Patientenakzeptanz.

Bevorzugte Ausgestaltungen der Erfindung sind in den Unteransprüchen angegeben.

Der Anteil an Mometasonfuorat in der erfindungsgemäßen Zusammensetzung kann auf einen Wert zwischen 0,01% und 0,2% eingestellt werden. Höhere in diesen Bereich fallende Mometasonfuoratkonzentrationen können zum Einsatz kommen in Zusammensetzungen, die zur Behandlung von schweren Ekzemformen wie z.B. dem chronischen Handekzem gedacht sind, niedrigere Konzentrationen bei der Therapie des atopischen Ekzems.

Besonders bevorzugt wird die erfindungsgemäße Zusammensetzung auf eine Mometasonfuoratkonzentration zwischen 0,05% und 0,15%, insbesondere auf 0,1 % eingestellt. Dieser Bereich deckt eine Vielzahl unterschiedlicher Applikationen ab.

Der Wassergehalt der erfindungsgemäßen Zusammensetzung kann zwischen 10% und 90% betragen. In diesem Bereich sind eine Vielzahl von unterschiedlichen galenischen Formulierungen möglich. Bevorzugt wird ein Wert zwischen 25 und 70% gewählt, womit ein für Emulsionen üblicher Bereich abgedeckt ist. Besonders bevorzugt wird die Zusammensetzung auf einen für Cremes und Salben typischen Wassergehalt zwischen 35% und 60% eingestellt.

In Abhängigkeit von dem Wassergehalt lässt sich die Zusammensetzung als Creme, Salbe, Emulsion etc. formulieren.

Bevorzugt werden im Rahmen der Erfindung als aromatische Alkohole Phenoxyethanol, Benzylalkohol, Phenoxyisopropanol und/oder Phenylethylalkohol eingesetzt. Es handelt sich dabei um erprobte pharmazeutische Substanzen, die wie auch die weiteren in der Kombination eingesetzten Lösungsmittel, eine gute Hautverträglichkeit aufweisen.

Die aromatischen Alkohole sind in der erfindungsgemäßen Zusammensetzung bevorzugt in einer Konzentration zwischen 0,1% und 10% enthalten. Alkoholkonzentrationen in diesem Bereich ermöglichen eine Anpassung bzw. Optimierung vieler unterschiedlicher Produkte. Insbesondere sind die aromatischen Alkohole zwischen 0,5% und 5%, besonders bevorzugt zwischen 1% und 3% enthalten. Die letztgenannten Bereiche sind für die meisten Rezepturen, insbesondere typische Cremes und Lotionen geeignet.

Die erfindungsgemäß in der Zusammensetzung vorgesehen Kombination enthält bevorzugt als Lösungsmittel der ersten Gruppe ethoxylierte Fettalkohole, Zuckeralkoholfettsäureester, ethoxylierte Zuckeralkoholfettsäureester und ethoxilierte Mono-, Di- oder Triglycerinester von gesättigten oder ungesättigten Fettsäuren sind.

Grundsätzlich geeignete Vertreter für Lösungsmittel der ersten Gruppe sind nachfolgend aufgeführt. Jeweils in Klammern sind die jeweiligen INCI-Bezeichnungen (International verbindliche Namensbezeichnungen für kosmetische Zutaten) der Lösungsmittel angegeben.

Geeignete Vertreter der in der ersten Gruppe genannten alkoxilierten Fettalkohole mit einer Kettenlänge von C8 - C22 sind z.B. Polyoxyethylen-Laurylether (Laureth-4, Laureth-10), Polyoxyethylen-Cetylether (Ceteth-20), Polyoxyethylen-Cetostearylether (Ceteareth-6, Ceteareth-25), Polyoxyethylen-Stearylether (Steareth-2, Steareth-21), PolyoxyethylenBehenylether (Beheneth-10), Polyoxypropylen-Stearylether (Polypropylenglykol-15 Stearyl Ether). Geeignete alkoxilierte Fettsäuren mit einer Kettenlänge von C8 - C22 sind z.B. Polyethylenglykol-8 Caprylat (Polyethylenglykol-8 Caprylate), Polyethylenglykol-8 Laurat (Polyethylenglykol-8 Laurate), Polyethylenglykol-8 Oleat (Polyethylenglykol-8 Oleate) und Polyethylenglykol-Stearat (Polyethylenglykol-40 Stearate, Polyethylenglykol-100 Stearate). Nicht alkoxilierte Fettsäuren mit einer Kettenlänge von C8 - C22 sind z.B. Palmitinsäure (Palmitic Acid), Stearinsäure (Stearic Acid) und Ölsäure (Oleic Acid), während nicht alkoxilierte Carbonsäureester mit Carbonsäuren einer Kettenlänge von C6 - C22 Isopropylmyrsitat (Isopropyl Myristate), Isopropylpalmitat (Isopropyl Palmitate), Diisopropyladipat (Diisopxopyl Adipate), Propylenglycolcaprylat (Propylene Glycol Caprylate), Propylenglycollaurat (Propylene Glycol Laurate), Propylenglycolstearat ((Propylene Glycol Stearate), Butylenglycol Dicaprylat/Dicaprat (Butylene Glycol Dicaprylate/Dicaprate), mittelkettige Triglyceride (Caprylic/Capric Triglyceride), Glycerolmonostearat (Glyceryl Stearate), Sorbitanlaurat (Sorbitan Laurate), Sorbitanoleat (Sorbitan Oleate) oder Sorbitanstearat (Sorbitan Stearate) umfassen können.

Die Konzentration des bzw. der aus der ersten Gruppe ausgewählten Lösungsmittel liegt vorteilhafterweise zwischen 1% und 40%. In diesem Bereich ist eine Anpassung an bzw. Optimierung für eine Vielzahl von Produkten möglich. Insbesondere wird ein Wert zwischen 2% und 30% gewählt, was übliche für Cremes und Lotionen geeignete Werte zur Sicherstellung der Löslichkeit des Mometasonfuroats abdeckt. Besonders bevorzugt sind Werte zwischen 2% und 15%, da Werte in diesem Bereich einen guten Kompromiß zwischen der Erhaltung der Löslichkeit des Wirkstoffs einerseits und der Emulsionsstabilität andererseits darstellen

Die Konzentration des Hexylenglykols liegt vorteilhafterweise zwischen 1% und 40%. In diesem Bereich ist eine Anpassung an bzw. Optimierung für eine Vielzahl von Produkten möglich. Insbesondere wird ein Wert zwischen 2% und 30% gewählt, was übliche für Cremes und Lotionen geeignete Werte zur Sicherstellung der Löslichkeit des Mometasonfuroats abdeckt. Besonders bevorzugt sind Werte zwischen 2% und 15%, da Werte in diesem Bereich einen guten Kompromiß zwischen der Erhaltung der Löslichkeit des Wirkstoffs einerseits und der Emulsionsstabilität andererseits darstellen.

Die Erfindung deckt nicht nur die bislang beschriebenen Zusammensetzungen ab, sondern umfasst auch die Verwendung der Kombination eines aromatischen Alkohols mit mindestens einem Lösungsmittel aus der ersten Gruppe und Hexylenglykol zur Verbesserung des Lösungsverhaltens von Mometasonfuroat in Gegenwart von Wasser. Durch Zusatz der erwähnten Kombination zu Zusammensetzungen, die zwischen 0,01% und 0,2% (w/w) Mometasonfuroat und Gegenwart von 10%-90% (w/w) Wasser aufweisen, wird sichergestellt, dass das Mometasonfuroat in Lösung bleibt und nicht ausfällt.

Die erfindungsgemäße Zusammensetzung kann als Lösung, Mikroemulsion, Gel, Lotion, Creme oder Salbe vorliegen.

Im folgenden sollen einige Beispiele für erfindungsgemäße Zusammensetzungen gegeben werden. Die Angaben sind jeweils Prozentangaben (w/w). Die Herstellung der jeweiligen Produkte auf Basis der angegebenen Zusammensetzungen beginnt in der Regel damit, dass das Mometasonfuroat in der erfindungsgemäß eingesetzten, mindestens einen aromatischen Alkohol und mindestens ein Lösungsmittel und Hexylenglykol enthaltenden Kombination aufgelöst wird und nachfolgend im Rahmen der Standardverfahren für diese Art von Produkten eingearbeitet wird. Die Verfahren sind nicht näher erläutert, da es sich dabei um herkömmliche dem Fachmann vertraute Verfahren handelt.

**Beispiel 1:Zusammensetzung für eine Emulsion**

| | |
|---|---|
| Mometasonfuroat | 0,02 |
| Phenoxyethanol | 1,00 |
| Steareth-21 | 4,00 |
| Hexylenglycol | 10,00 |
| Paraffinum Liquidum | 10,00 |
| Natriumcitrat Dihydrat | 0,32 |
| Citronensäure, wasserfrei | 0,42 |
| Wasser, gereinigt | 74,24 |

**Beispiel 2: Zusammensetzung für eine Lösung**

| | |
|---|---|
| Mometasonfuroat | 0,20 |
| Phenoxyethanol | 2,00 |
| Diethylenglycolmonoethylether | 40,00 |
| Hexylenglycol | 20,00 |
| Natriumcitrat Dihydrat | 0,32 |
| Citronensäure, wasserfrei | 0,42 |
| Wasser, gereinigt | 37,16 |

**Beispiel 3: Zusammensetzung für eine Lotion (Bezugsbeispiel)**

| | |
|---|---|
| Mometasonfuroat | 0,10 |
| Phenoxyethanol | 2,00 |
| Polysorbat 40 | 4,50 |
| Paraffinum Liquidum | 20,00 |
| Diethylenglycolmonoethylaether | 10,00 |
| Citronensäure, wasserfrei | 0,42 |
| Natriumcitrat Dihydrat | 0,32 |
| Methylparaben | 0,30 |
| Xanthan Gum | 0,30 |
| Wasser, gereinigt | 62,06 |

**Beispiel 4: Zusammensetzung für eine Mikroemulsion (Bezugsbeispiel)**

| | |
|---|---|
| Mometasonfuroat | 0,10 |
| Ölsäure | 5,00 |
| Polysorbat 80 | 8,00 |
| Propylenglycol | 17,50 |
| Hexylenglycol | 10,00 |
| Poloxamer 331 | 12,00 |
| Wasser, gereinigt | 47,40 |

**Beispiel 5: Zusammensetzung für eine Creme**

| | |
|---|---|
| Mometasonfuroat | 0,10 |
| Benzylalkohol | 2,00 |
| Ceteareth-20 | 1,00 |
| Glycerohnonostearat / Polyethylenglykol-30 Stearat (Arlatone 983 S) | 4,00 |
| Mittelkettige Triglyceride | 4,00 |
| Weißes Vaselin | 10,00 |
| Paraffinum Liquidum | 7,00 |
| Cetylalkohol | 3,00 |
| Hexylenglycol | 10,00 |
| Citronensäure, wasserfrei | 0,42 |
| Natriumcitrat Dihydrat | 0,32 |
| Wasser, gereinigt | 58,16 |

**Beispiel 6: Zusammensetzung für eine Creme (Bezugsbeispiel)**

| | |
|---|---|
| Mometasonfuroat | 0,10 |
| Benzylalkohol | 3,00 |
| Ceteareth-20 | 3,00 |
| Mittelkettige Triglyceride | 6,00 |
| Weißes Vaselin | 6,00 |
| Cetylalkohol | 2,00 |
| Propylenglycol | 10,00 |
| Citronensäure, wasserfrei | 0,42 |
| Natriumcitrat Dihydrat | 0,32 |
| Methylparaben | 0,20 |
| Wasser, gereinigt | 68,96 |

**Beispiel 7: Zusammensetzung für eine Creme**

| | |
|---|---|
| Mometasonfuroat | 0,10 |
| Phenoxyethanol | 2,00 |
| Polysorbat 20 | 5,00 |
| Propylenglycollaurat | 10,00 |
| Glycerolmonostearat | 2,00 |
| Propylenglycolstearat | 1,00 |
| Weißes Vaselin | 10,00 |
| Cetylalkohol | 6,00 |
| Hexylenglycol | 10,00 |
| Citronensäure, wasserfrei | 0,42 |
| Natriumcitrat Dihydrat | 0,32 |
| Wasser, gereinigt | 53,16 |

**Beispiel 8: Zusammensetzung für eine Mikroemulsion (Bezugsbeispiel)**

| | |
|---|---|
| Mometasonfuroat | 0,10 |
| Phenoxyethanol | 1,00 |
| Polyethylenglykol-6 Caprylic/Capric Glyceride | 7,00 |
| Propylenglycollaurat | 10,00 |
| Polyethylenglykol-18 Castor Oil | 12,00 |
| Paraffinum Liquidum | 10,00 |
| Sodium Laureth Sulfate | 24,00 |
| Cocamidopropyl Betaine | 6,00 |
| Sodium Chloride | 2,00 |
| Citronensäure, wasserfrei | 0,10 |
| Wasser, gereinigt | 37,80 |

**Beispiel 9: Zusammensetzung für eine Lotion**

| | |
|---|---|
| Mometasonfuroat | 0,10 |
| Phenoxyethanol | 1,00 |
| Steareth-21 | 6,00 |
| Mittelkettige Triglyceride | 6,00 |
| Diisopropyl Adipate | 3,00 |
| Propylenglycolcaprylat | 5,00 |
| Paraffinum Liquidum | 13,48 |
| Hexylenglycol | 10,00 |
| Citronensäure, wasserfrei | 0,42 |
| Natriumcitrat Dihydrat | 0,32 |
| Methylparaben | 0,30 |
| Wasser, gereinigt | 50,38 |

**Beispiel 10: Zusammensetzung für eine Creme**

| | |
|---|---|
| Mometasonfuroat | 0,10 |
| Phenoxyethanol | 1,00 |
| Steareth-21 | 5,00 |
| Steareth-2 | 5,00 |
| Mittelkettige Triglyceride | 6,50 |
| Diisopropyl Adipate | 3,00 |
| Propylenglycolcaprylat | 5,00 |
| Weißes Vaselin | 10,00 |
| Paraffinum Liquidum | 7,00 |
| Cetylalkohol | 6,00 |
| Hexylenglycol | 10,00 |
| Citronensäure, wasserfrei | 0,42 |
| Natriumcitrat Dihydrat | 0,32 |
| Xanthan Gum | 0,10 |
| Wasser, gereinigt | 40,56 |

**Beispiel 11: Zusammensetzung für eine Creme (Bezugsbeispiel)**

| | |
|---|---|
| Mometasonfuroat | 0,10 |
| Phenoxyethanol | 1,00 |
| Steareth-21 | 5,00 |
| Steareth-2 | 5,00 |
| Mittelkettige Triglyceride | 6,50 |
| Diisopropyl Adipate | 3,00 |
| Propylenglycolcaprylat | 5,00 |
| Weißes Vaselin | 10,00 |
| Paraffinum Liquidum | 7,00 |
| Cetylalkohol | 6,00 |
| Diethylenglycolmonoethylether | 10,00 |
| Citronensäure, wasserfrei | 0,42 |
| Natriumcitrat Dihydrat | 0,32 |
| Xanthan Gum | 0,10 |
| Wasser, gereinigt | 40,56 |

**Beispiel 12: Zusammensetzung für eine Salbe**

| | |
|---|---|
| Mometasonfuroat | 0,05 |
| Benzylalkohol | 2,00 |
| Hexylenglycol | 10,00 |
| Paraffinum Liquidum | 20,00 |
| Sorbitansesquioleat | 10,00 |
| Paraffinum durum | 3,00 |
| Gebleichtes Wachs | 8,00 |
| Nlikrokristallines Wachs | 1,00 |
| Magnesiumsulfat Heptahydrat | 0,70 |
| Wasser, gereinigt | 45,25 |

## Patentansprüche

1. Auf die Haut applizierbare pharmazeutische Zusammensetzung, enthaltend
a) 0,01 % - 0,2% (w/w) Mometasonfuroat,
b) 10% - 90% (w/w) Wasser und
c) eine Kombination von
i) mindestens einem aromatischen Alkohol; und
ii) mindestens einem Lösungsmittel ausgewählt aus einer ersten Gruppe, zu der alkoxilierte Fettalkohole mit einer Kettenlänge von C8-C22, alkoxilierte und nicht alkoxilierte Fettsäuren mit einer Kettenlänge von C8-C22 und alkoxilierte und nicht alkoxilierte Carbonsäureester mit Carbonsäuren einer Kettenlänge von C6-C22 gehören; und
iii) Hexylenglykol;
sowie
d) gegebenenfalls weitere Zusatzstoffe.

2. Zusammensetzung nach Anspruch 1, **dadurch gekennzeichnet, dass** der Anteil an Mometasonfuorat zwischen 0,05% und 0,15%, insbesondere 0,1 % beträgt.

3. Zusammensetzung nach Anspruch 1, **dadurch gekennzeichnet, dass** der Wassergehalt zwischen 25% und 70% beträgt.

4. Zusammensetzung nach Anspruch 3, **dadurch gekennzeichnet, dass** der Wassergehalt zwischen 35% und 60% beträgt.

5. Zusammensetzung nach Anspruch 1, **dadurch gekennzeichnet, dass** der Anteil an aromatischem Alkohol zwischen 0,1% und 10% beträgt.

6. Zusammensetzung nach Anspruch 5, **dadurch gekennzeichnet, dass** der Anteil an aromatischem Alkohol zwischen 0,5% und 5 %, beträgt.

7. Zusammensetzung nach Anspruch 6, **dadurch gekennzeichnet, dass** der Anteil an aromatischem Alkohol zwischen 1 % und 3 %, beträgt.

8. Zusammensetzung nach Anspruch 1, **dadurch gekennzeichnet, dass** der Anteil des bzw. der aus der ersten Gruppe ausgewählten Lösungsmittel zwischen 1 % und 40% beträgt.

9. Zusammensetzung nach Anspruch 8, **dadurch gekennzeichnet, dass** der Anteil des bzw. der aus der ersten Gruppe ausgewählten Lösungsmittel zwischen 2% und 30% beträgt.

10. Zusammensetzung nach Anspruch 9, **dadurch gekennzeichnet, dass** der Anteil des bzw. der aus der ersten Gruppe ausgewählten Lösungsmittel zwischen 2% und 15% beträgt.

11. Zusammensetzung nach Anspruch 1, **dadurch gekennzeichnet, dass** der Anteil des Hexylenglykols zwischen 1 % und 40% beträgt.

12. Zusammensetzung nach Anspruch 11, **dadurch gekennzeichnet, dass** der Anteil des Hexylenglykols zwischen 2% und 30% beträgt.

13. Zusammensetzung nach Anspruch 12, **dadurch gekennzeichnet, dass** der Anteil des Hexylenglykols zwischen 2% und 15% beträgt.

14. Zusammensetzung nach Anspruch 1, **dadurch gekennzeichnet, dass** der aromatische Alkohol Phenoxyethanol, Benzylalkohol, Phenoxyisopropanol und/oder Phenylethylalkohol ist.

15. Zusammensetzung nach Anspruch 1, **dadurch gekennzeichnet, dass** die Lösungsmittel der ersten Gruppe ethoxylierte Fettalkohole, Zuckeralkoholfettsäureester, ethoxylierte Zuckeralkoholfettsäureester und ethoxilierte Mono-, Di- oder Triglycerinester von gesättigten oder ungesättigten Fettsäuren sind.

16. Zusammensetzung nach Anspruch 1, **dadurch gekennzeichnet, dass** die Carbonsäureester der ersten Gruppe Diisopropyladipat, Propylenglycolcaprylat und/oder Propylenglycollaurat sind.

17. Zusammensetzung nach Anspruch 1, **dadurch gekennzeichnet, dass** sie als Lösung, Mikroemulison, Gel, Lotion, Creme oder Salbe vorliegt.

18. Verwendung einer Kombination von i) mindestens einem aromatischen Alkohol, und ii) mindestens einem Lösungsmittel ausgewählt aus der ersten im Anspruch 1 definierten Gruppe, und iii) Hexylenglykol zur Verbesserung des Lösungsverhalten von Mometasonfuroat in Gegenwart von Wasser.

## Claims

1. A pharmaceutical composition for application on the skin, containing
a) 0.01 % - 0.2% (w/w) mometasone furoate,
b) 10% - 90% (w/w) water and
c) a combination of
i) at least one aromatic alcohol; and
ii) at least one solvent selected from
a first group, which includes alkoxylated fatty alcohols with a chain length of C8 - C22, alkoxylated and non-alkoxylated fatty acids with a chain length of C8 - C22 and alkoxylated and non-alkoxylated carboxylic acid esters with carboxylic acids having a chain length of C6 - C22; and
iii) hexylene glycol;
and
d) optionally other additives.

2. The composition according to claim 1, **characterised in that** the proportion of mometasone furoate is between 0.05% and 0.15%, in particular 0.1%.

3. The composition according to claim 1, **characterised in that** the water content is between 25% and 70%.

4. The composition according to claim 3, **characterised in that** the water content is between 35% and 60%.

5. The composition according to claim 1, **characterised in that** the proportion of aromatic alcohol is between 0.1% and 10%.

6. The composition according to claim 5, **characterised in that** the proportion of aromatic alcohol is between 0.5% and 5%.

7. The composition according to claim 6, **characterised in that** the proportion of aromatic alcohol is between 1% and 3%.

8. The composition according to claim 1, **characterised in that** the proportion of the solvent or solvents selected from the first group is between 1% and 40%.

9. The composition according to claim 8, **characterised in that** the proportion of the solvent or solvents selected from the first group is between 2% and 30%.

10. The composition according to claim 9, **characterised in that** the proportion of the solvent or solvents selected from the first group is between 2% and 15%.

11. The composition according to claim 1, **characterised in that** the proportion of hexylene glycol is between 1% and 40%.

12. The composition according to claim 11, **characterised in that** the proportion of hexylene glycol is between 2% and 30%.

13. The composition according to claim 12, **characterised in that** the proportion of hexylene glycol is between 2% and 15%.

14. The composition according to claim 1, **characterised in that** the aromatic alcohol is phenoxyethanol, benzyl alcohol, phenoxyisopropanol and/or phenylethyl alcohol.

15. The composition according to claim 1, **characterised in that** the solvents from the first group are ethoxylated fatty alcohols, sugar alcohol fatty acid esters, ethoxylated sugar alcohol fatty acid esters and ethoxylated mono-, di- or triglycerol esters of saturated or unsaturated fatty acids.

16. The composition according to claim 1, **characterised in that** the carboxylic acid esters from the first group are diisopropyl adipate, propylene glycol caprylate and/or propylene glycol laurate.

17. The composition according to claim 1, **characterised in that** it is present as a solution, microemulsion, gel, lotion, cream or ointment.

18. Use of a combination of i) at least one aromatic alcohol and ii) at least one solvent selected from the first group defined in claim 1 and iii) hexylene glycol to improve the solution behaviour of mometasone furoate in the presence of water.

## Revendications

1. Composition pharmaceutique pouvant être appliquée sur la peau, contenant
a) 0,01 % à 0,2 % (en masse) de furoate de mométasone
b) 10 % à 90 % (en masse) d'eau et
c) une combinaison de
i) au moins un alcool aromatique ; et
ii) au moins un solvant, choisi parmi
un premier groupe, auquel appartiennent des alcools gras alcoxylés d'une longueur de chaîne de C8 à C22, des acides gras alcoxylés et non alcoxylés d'une longueur de chaîne de C8 à C22, et des esters d'acides carboxyliques alcoxylés et non alcoxylés avec des acides carboxyliques d'une longueur de chaîne de C6 à C22, et
iii) de l'hexylène glycol
ainsi que
d) le cas échéant, d'autres additifs.

2. Composition selon la revendication 1, **caractérisée en ce que** la proportion de furoate de mométasone est comprise entre 0,05 % et 0,15 %, en particulier est égale à 0,1 %.

3. Composition selon la revendication 1, **caractérisée en ce que** la teneur en eau est comprise entre 25 % et 70 %.

4. Composition selon la revendication 3, **caractérisée en ce que** la teneur en eau est comprise entre 35 % et 60 %.

5. Composition selon la revendication 1, **caractérisée en ce que** la teneur en alcool aromatique est comprise entre 0,1 % et 10 %.

6. Composition selon la revendication 5, **caractérisée en ce que** la teneur en alcool aromatique est comprise entre 0,5 % et 5 %.

7. Composition selon la revendication 6, **caractérisée en ce que** la teneur en alcool aromatique est comprise entre 1 % et 3 %.

8. Composition selon la revendication 1, **caractérisée en ce que** la teneur en le ou les solvants choisis parmi le premier groupe est comprise entre 1 % et 40 %.

9. Composition selon la revendication 8, **caractérisée en ce que** la teneur en le ou les solvants choisis parmi le premier groupe est comprise entre 2 % et 30 %.

10. Composition selon la revendication 9, **caractérisée en ce que** la teneur en le ou les solvants choisis parmi le premier groupe est comprise entre 2 % et 15 %.

11. Composition selon la revendication 1, **caractérisée en ce que** la teneur en hexylène glycol est comprise entre 1 % et 40 %.

12. Composition selon la revendication 11, **caractérisée en ce que** la teneur en hexylène glycol est comprise entre 2 % et 30 %.

13. Composition selon la revendication 12-, **caractérisée en ce que** la teneur en hexylène glycol est comprise entre 2 % et 15 %.

14. Composition selon la revendication 1, **caractérisée en ce que** l'alcool aromatique est du phénoxyéthanol, de l'alcool benzylique, du phénoxyisopropanol et/ou de l'alcool phényléthylique.

15. Composition selon la revendication 1, **caractérisée en ce que** les solvants du premier groupe sont des acides gras éthoxylés, des esters d'alditols et d'acides gras, des esters d'alditols et d'acides gras éthoxylés et des mono-, di- ou triesters de glycérol et d'acides gras saturés ou insaturés.

16. Composition selon la revendication 1, **caractérisée en ce que** les esters d'acides carboxyliques du premier groupe sont l'adipate de diisopropyle, le caprylate de propylène glycol et/ou le laurate de propylène glycol.

17. Composition selon la revendication 1, **caractérisée en ce qu'**elle se présente comme solution, micro-émulsion, gel, lotion, crème ou pommade.

18. Utilisation d'une combinaison de i) au moins un alcool aromatique ; de ii) au moins un solvant choisi parmi un premier groupe défini à la revendication 1, et de iii) l'hexylène glycol pour l'amélioration du comportement de dissolution du furoate de mométasone en présence d'eau.
